# EUROPÄISCHE PATENTANMELDUNG

(11) **EP 4 781 921 A1**
(43) Veröffentlichungstag der Anmeldung: **29.07.2026**
(21) Anmeldenummer: 25153545.6
(22) Anmeldetag: 23.01.2025
(51) Int. Cl.: A61B 10/02, A61B 17/00, A61B 17/3203

(54) **VORRICHTUNG, ANORDNUNG UND VERFAHREN ZUM ABTRAGEN VON GEWEBEPARTIKELN**

(71) Anmelder: Erbe Elektromedizin GmbH, 72072 Tübingen (DE)
(72) Erfinder: Straub, Frank, 72770 Reutlingen (DE); Salkic, Nermin, 72116 Moessingen (DE); Fech, Andréas, 72072 Tuebingen, (DE)
(74) Vertreter: Rüger Abel Patentanwälte PartGmbB

(57) **Zusammenfassung**

Die Erfindung betrifft eine Vorrichtung 15 zum Abtragen von Gewebepartikeln P eines Gewebes G von der Gewebeoberfläche GO oder einem oberflächennahen Gewebebereich, insbesondere ohne Blutungen zu verursachen. Dadurch können Gewebepartikel P bzw. Zellen gesammelt werden, beispielsweise für eine spätere Gewebeanalyse. Die Vorrichtung 15 hat hierzu ein Kopfteil 16, das einen Innenraum 24 begrenzt, in den ein Düsenrohr 26 hineinragt. Das Düsenrohr hat mehrere benachbart zueinander angeordnete Düsenöffnungen 30, die zu einer Kopfteilöffnung 25 des Kopfteils 16 hin ausgerichtet sind. Benachbart zur Kopfteilöffnung 25 hat das Kopfteil 16 eine Auflagefläche 17, die zur Auflage auf der Gewebeoberfläche GO eingerichtet ist. Über einen Zufuhrkanal 27 kann unter Druck stehende Flüssigkeit in das Düsenrohr 30 eingeleitet und unter Bildung von kompakten Fluidstrahlen aus den Düsenöffnungen 30 ausgestoßen werden, um beim Auftreffen auf der Gewebeoberfläche GO Gewebepartikel P abzulösen, die sich dann im Innenraum 24 des Kopfteils 16 befinden. Der Innenraum 24 ist fluidisch mit einem Absaugkanal 34 verbunden. Über den Absaugkanal kann eine Absaugströmung S Gewebepartikel aus dem Innenraum 24 absaugen und in einer Partikelsammeleinrichtung 35 sammeln, die fluidisch mit dem Absaugkanal 34 verbindbar ist. Mittels einer solchen Vorrichtung 15 kann schonend eine Gewebeprobe in Form von Gewebepartikeln P entnommen werden. Die Vorrichtung 15 kann in Kombination mit einem Endoskop 20 eingesetzt werden.

## Beschreibung

Die Erfindung betrifft eine Vorrichtung, eine Anordnung und ein Verfahren zum Ablösen von Gewebepartikeln von einer Gewebeoberfläche, wodurch beispielsweise Zellen abgelöst oder abgetragen und entnommen werden können, insbesondere als Gewebeprobe für eine weitere pathologische Analyse. Zusätzlich oder alternativ kann die Erfindung auch zur Gewebereinigung oder Gewebebehandlung an der Gewebeoberfläche verwendet werden. Die Erfindung ist insbesondere geeignet zum Ablösen von Gewebepartikeln an Schleimhäuten, wie z.B. im Magen, im Darm, in der Speiseröhre, im Gallengang oder an anderen inneren Gewebeoberflächen eines menschlichen und/oder tierischen Körpers.

Das Entnehmen von oberflächennahen Gewebepartikeln oder Zellen kann beispielsweise angewandt werden, um die entnommenen Zellen zu untersuchen, beispielsweise um Zellveränderungen frühzeitig diagnostizieren zu können. Dabei kann es vorteilhaft oder notwendig sein, Gewebepartikel bzw. Zellen an größeren Bereichen der Gewebeoberfläche abzutragen bzw. zu entnehmen, damit lokal begrenzte Gewebe- oder Zellveränderungen mit einer gewissen Wahrscheinlichkeit aufgefunden werden können. Die Gewebepartikel müssen bei der Entnahme von der Gewebeoberfläche entfernt werden, um eine Kontamination anderer Flächenbereiche der Gewebeoberfläche zu vermeiden, auf die die Vorrichtung anschließend einwirkt. Außerdem müssen die entnommenen Gewebepartikel (insbesondere Zellen) einer Gewebeoberfläche oder einem Gewebeoberflächenbereich zugeordnet werden können, so dass die Untersuchung der Zellen der Gewebeoberfläche bzw. dem Gewebeoberflächenbereich zugeordnet werden kann, von dem sie entnommen wurden.

Es ist bekannt, Gewebeproben mittels eines Flüssigkeitsstrahls in einen Absaugkanal zu fördern, um diese einer Untersuchung zuzuführen. Entsprechende Instrumente sind aus der EP 4 072 448 A1 der EP 1 182 974 B1 der EP 2 019 628 Al, der EP 1 433 423 Al, der US 6 572 578 B1 und der EP 3 500 192 A1 bekannt. Diese Instrumente weisen jeweils einen Flüssigkeitskanal auf, der an einer Auslassöffnung einen Fluidstrahl erzeugt, der direkt auf die Saugöffnung eines Absaugkanals gerichtet ist.

Es sind auch Instrumente bekannt, bei denen ein Fluidstrahl direkt auf Gewebe geleitet wird, so zum Beispiel aus der EP 2 303 156 A2, der US 2021/0308484 A1 und der US 6 030 399 A. Letztgenannte Druckschrift dient der Entnahme von Blutproben aus der Haut.

Das aus der US 6 030 399 A bekannte Instrument weist einen Kopf auf, der einen Innenraum umschließt. Der Innenraum ist zu der Oberfläche der Haut hin offen, aus der Blut zu entnehmen ist. Der Rand der Öffnung ist auf die Haut aufzusetzen, wodurch der Innenraum nach außen hin abgedichtet wird. Um die Abdichtung möglichst vollständig sicherzustellen, ist der Rand der Öffnung mit einer entsprechenden Dichtung versehen. Ein Speisekanal dient zur Zuführung von Flüssigkeit in den Innenraum, wobei die Flüssigkeit als scharfer Strahl auf die Haut auftrifft und in diese einsticht. Auf diese Weise freigesetztes Blut oder Gemisch aus Blut und zugeführter Flüssigkeit wird über Absaugkanäle abgeführt, die mit dem Innenraum verbunden sind. Aus dem Fachartikel LightdaleCJ, Tiscornia-Wasserman P, Sethi A et al: "Endoscopy-Guided High-Pressure Spray "Power-Wash" to obtain Cytopathology For Detection Of Gastric Intestinal Metaplasia: A Proof Of Concept Study"; Gastrointest Endosc 2022; 95: AB457-AB458 ist bekannt, dass sich mit einem Strahl Zellen von einer Schleimhaut des Gastrointestinaltraktes abwaschen lassen.

US 5 037 431 A offenbart eine chirurgische Vorrichtung, mittels der ein Fluidstrahl erzeugt und auf eine Gewebeoberfläche gerichtet werden kann, um krankes Gewebe zu fragmentieren. An seinem distalen Ende hat das Instrument um die Fluiddüse eine kuppel- oder glockenförmige Abschirmung, die auf das zu behandelnde Gewebe aufgesetzt werden kann. Das Innere dieser Abschirmung ist außerdem mit einer Saugleitung zum Absaugen von Gewebefragmenten verbunden.

US 2016/199566 A1 beschreibt ein Handinstrument zum Reinigen von Wunden mittels eines Wasserstrahls. An einem hohlzylindrischen distalen Ende kann mittels einer Fluiddüse ein axialer Fluidstrahl erzeugt werden. Die Stirnseite des hohlzylindrischen distalen Endes kann auf das Gewebe aufgesetzt werden, wobei die Stirnfläche in einer Ebene rechtwinklig oder schräg zur Zylinderachse ausgerichtet sein kann. Bei einem anderen Ausführungsbeispiel ist die Fluiddüse von der Zylinderachse versetzt angeordnet, wobei der Fluidstrahl in der Ebene der Stirnfläche des distalen Endes am Schnittpunkt mit der Zylinderachse auf eine Gewebeoberfläche auftrifft.

Bei dem chirurgischen Instrument zur Gewebebehandlung gemäß US 2004/0243157 A1 ist am distalen Ende eine sphärische Abschirmung vorgesehen, die einen Innenraum begrenzt. Ein Absaugkanal mündet in diesen Innenraum ein. In einer axialen Richtung gegenüberliegend zu dem Absaugkanal ist eine Fluiddüse angeordnet, die einen Fluidstrahl durch den Innenraum in den Absaugkanal richten kann. Die Abschirmung ist an einer Seite offen, so dass Gewebeteile in den Innenraum der Abschirmung hineinragen und zwischen der Fluiddüse und dem Absaugkanal angeordnet werden können. Dabei trifft der Fluidstrahl auf die Gewebeteile auf und kann Teile hiervon entfernen, die dann direkt in den Absaugkanal gefördert werden.

Weitere ein Gewebe mit einem Fluidstrahl behandelnde Instrumente sind aus WO 03/096871 A2 und US 6 030 399 A bekannt.

Bei der Entnahme von Gewebeproben bzw. beim Abtragen oder Ablösen von Gewebepartikel von oberflächennahem Gewebe, beispielsweise um Metaplasien zu untersuchen, sollen möglichst keine blutenden Gewebeverletzungen entstehen. Bei der Erfindung geht es darum, eine Vorrichtung bzw. ein Verfahren zu schaffen, bei dem Gewebepartikel abgetragen bzw. entnommen werden, ohne die Gewebeoberfläche zu schädigen und insbesondere (soweit möglich) ohne blutende Gewebeverletzungen zu verursachen.

Es kann daher als Aufgabe der vorliegenden Erfindung angesehen werden, eine Vorrichtung zu schaffen, die eine schonende und insbesondere unblutige oberflächliche Zellgewinnung ermöglicht.

Diese Aufgabe wird mit einer Vorrichtung gemäß den Merkmalen des Patentanspruches 1, einer Anordnung mit den Merkmalen des Patentanspruches 15 und einem Verfahren mit den Merkmalen des Patentanspruches 16 gelöst.

Die erfindungsgemäße Vorrichtung ist dazu eingerichtet, Gewebepartikel - insbesondere Gewebezellen - von einem Gewebe und vorzugsweise von einer Gewebeoberfläche abzutragen. Dadurch können Zellproben bzw. Gewebepartikelproben gewonnen werden, die dann beispielsweise für eine Untersuchung gesammelt werden können. Die Vorrichtung ist dabei dazu eingerichtet, das Gewebe bzw. die Gewebeoberfläche nicht mehr als notwendig zu beeinträchtigen und insbesondere blutende Verletzungen zu vermeiden. Die Gewebepartikel oder Zellen können von einem menschlichen oder tierischen Körper entnommen werden. Beispielsweise kann es sich bei dem Gewebe um eine Schleimhaut handeln - zum Beispiel im Magen, im Darm, in der Speiseröhre oder im Gallengang - oder um eine andere innere Gewebeoberfläche.

Die Vorrichtung verwendet mehrere Flüssigkeitsstrahlen, die auf das Gewebe bzw. die Gewebeoberfläche gerichtet werden und dort auftreffen. Durch das Auftreffen werden Gewebepartikel bzw. Zellen abgelöst, die anschließend mittels der Vorrichtung abgesaugt und in einer Partikelsammeleinrichtung gesammelt werden können. Als Flüssigkeit zur Erzeugung der Flüssigkeitsstrahlen kann beispielsweise Wasser oder eine physiologische Kochsalzlösung verwendet werden.

Zu diesem Zweck hat die Vorrichtung ein Kopfteil mit einem Innenraum, der mittels einer Kopfteilöffnung von außen zugänglich ist. Abgesehen von der Kopfteilöffnung kann das Kopfteil den Innenraum ansonsten von der unmittelbaren Umgebung abtrennen. Optional können zusätzlich zu der Kopfteilöffnung eine oder mehrere Belüftungsöffnungen den Innenraum mit der unmittelbaren Umgebung des Kopfteils fluidisch verbinden, um eine Gaseinströmung in den Innenraum zu ermöglichen.

Das Kopfteil hat eine Auflagefläche, die dazu eingerichtet ist, auf dem Gewebe bzw. der Gewebeoberfläche während der Verwendung der Vorrichtung angeordnet zu werden. Die Auflagefläche ist benachbart zur Kopfteilöffnung angeordnet und kann unmittelbar an die Kopfteilöffnung anschließen. Bei einem Ausführungsbeispiel kann die Auflagefläche zumindest auf zwei der Kopfteilöffnung gegenüberliegenden Seiten des Kopfteils vorhanden sein. Die Auflagefläche kann die Kopfteilöffnung insbesondere an zwei oder mehr Seiten begrenzen und beispielsweise vollständig umschlie-ßen. Bei einem Ausführungsbeispiel kann sich die Auflagefläche in einer Ebene erstrecken, die parallel zu einer Längsrichtung und parallel zu einer Querrichtung ausgerichtet ist. Alternativ kann die Auflagefläche auch entlang einer virtuellen gekrümmten Fläche, insbesondere einer virtuellen Zylindermantelfläche ausgerichtet sein. Diese gekrümmte virtuelle Fläche ist vorzugsweise in Querrichtung gekrümmt und in Längsrichtung gerade.

An dem Kopfteil hat die Vorrichtung ein Düsenrohr und/oder einen Düsenschlauch. Das Düsenrohr und/oder der Düsenschlauch erstreckt sich entlang der Längsachse mit Abstand zur Kopfteilöffnung in den Innenraum hinein. An dem Düsenrohr und/oder dem Düsenschlauch sind mehrere Düsenöffnungen vorhanden. Es ist bevorzugt, wenn die Düsenöffnungen entlang einer Geraden angeordnet sind, die parallel zur Längsachse ausgerichtet ist. Die Düsenöffnungen sind am Düsenrohr und/oder Düsenschlauch auf der Seite vorhanden, die der Kopfteilöffnung zugewandt ist. Die Düsenöffnungen sind zur Kopfteilöffnung hin ausgerichtet.

Die Düsenöffnungen sind vorzugsweise länglich oder schlitzförmig und haben in Längsrichtung eine größere Dimension (Länge) verglichen mit ihrer Dimension in Querrichtung (Breite).

Mit dem Inneren des Düsenrohrs bzw. Düsenschlauchs und somit den Düsenöffnungen ist ein Zufuhrkanal fluidisch verbunden. Über den Zufuhrkanal ist das Düsenrohr bzw. der Düsenschlauch fluidisch mit einer Flüssigkeitsquelle verbunden oder verbindbar. Somit kann über den Zufuhrkanal unter Druck stehende Flüssigkeit in das Düsenrohr und/oder in den Düsenschlauch eingeleitet und durch die Düsenöffnungen des Düsenrohrs bzw. Düsenschlauchs ausgestoßen werden.

Jede Düsenöffnung ist dazu eingerichtet, einen vorzugsweise kompakten, zusammenhängenden Flüssigkeitsstrahl zu formen, wenn dem Düsenrohr und/oder dem Düsenschlauch Flüssigkeit unter Druck zugeführt wird. Dieser Flüssigkeitsstrahl tritt an der betreffenden Düsenöffnung aus, durchquert einen Teil des Innenraums des Kopfteils und kann dann durch die Kopfteilöffnung auf benachbart dazu angeordnetes Gewebe bzw. auf die Gewebeoberfläche auftreffen, insbesondere wenn die Auflagefläche des Kopfteils am Gewebe anliegt. Der Flüssigkeitsdruck im Düsenrohr bzw. Düsenschlauch und/oder an der Flüssigkeitsquelle kann beispielsweise mindestens 1,0 bis 80 bar und vorzugsweise maximal 10 bar betragen. Die aus den Düsenöffnungen austretende Flüssigkeit ist somit nicht zerstäubt.

Die Düsenöffnungen sind vorzugsweise derart ausgerichtet, dass die Flüssigkeitsstrahlen rechtwinklig zur Längsachse ausgerichtet und weiter vorzugsweise rechtwinkelig zur Querrichtung ausgerichtet. Die Düsenöffnungen sind vorzugsweise derart ausgerichtet, dass die Flüssigkeitsstrahlen parallel zueinander ausgerichtet und liegen insbesondere in einer gemeinsamen Ebene, die die Längsachse enthält. Die Flüssigkeitsstrahlen durchsetzen die Ebene der Kopfteilöffnung bevorzugt rechtwinklig. Vorzugsweise kann die Ausrichtung Flüssigkeitsstrahlen gegenüber der Längsrichtung und/oder Querrichtung in einem Winkelbereich von einschließlich 85° bis einschließlich 95° oder von einschließlich 87° bis einschließlich 93° oder von einschließlich 89° bis einschließlich 91° liegen.

In den Innenraum mündet außerdem ein Absaugkanal. Der Absaugkanal ist also fluidisch mit dem Innenraum verbunden. Wenn in dem Absaugkanal eine Saugströmung erzeugt wird, beispielsweise indem der Absaugkanal an eine Saugeinheit oder Unterdruckeinheit angeschlossen wird, können die durch Flüssigkeitsstrahlen vom Gewebe abgetragene Gewebepartikel bzw. Gewebezellen aus dem Innenraum abgesaugt und einer Partikelsammeleinrichtung zugeleitet werden. Die Gewebepartikel können dabei mittels eines Fluidstroms im Absaugkanal gefördert werden. In diesem Fluidstrom oder Absaugstrom können auch ein Gas und/oder eine Flüssigkeit enthalten sein, zum Beispiel Teile der von der Vorrichtung ausgestoßenen Flüssigkeit, Gewebeflüssigkeit, Luft oder Gas aus der Umgebung des Kopfteils.

Mittels der mehreren Flüssigkeitsstrahlen können Gewebepartikel bzw. Gewebezellen gleichzeitig an mehreren Stellen des Gewebes abgetragen und auf diese Weise gesammelt werden. Wenn das Kopfteil über eine Gewebeoberfläche bewegt wird, lassen sich Gewebeproben von größeren Gewebebereichen schnell und effizient sammeln. Dies kann beispielsweise von Bedeutung sein, wenn in einem tierischen oder menschlichen Körper größere Gewebeareale auf Gewebe- bzw. Zellveränderungen untersucht werden sollen und hierfür entlang des gesamten Gewebeareals an mehreren verschiedenen Stellen jeweils Gewebeproben entnommen werden sollen.

Vorzugsweise mündet der Absaugkanal benachbart zum Düsenrohr und/oder Düsenschlauch in den Innenraum. Der Absaugkanal kann eine oder mehrere Kanalmündungen aufweisen. Beispielsweise können mehrere Kanalmündungen des Absaugkanals in einer Umfangsrichtung um die Längsachse verteilt um das Düsenrohr und/oder den Düsenschlauch angeordnet sein. Die wenigstens eine Kanalmündung ist beispielsweise in einer Ebene rechtwinkelig oder schräg zur Längsrichtung ausgerichtet. Im Bereich der Kanalmündungen ist die Absaugströmung zumindest im Wesentlichen parallel zur Längsachse ausgerichtet.

Die Vorrichtung hat außerdem eine Außenhülle, die auch als Schaft bezeichnet werden kann. Die Außenhülle ist vorzugsweise flexibel biegbar. Die Außenhülle ist vorzugsweise unmittelbar mit dem Kopfteil verbunden. Alternativ können zwischen dem Kopfteil und der Außenhülle auch individuelle Verbindungsteile vorhanden sein. Die Außenhülle ist beim bevorzugten Ausführungsbeispiel ein quer zu ihrer Erstreckungsrichtung biegbarer Schlauch. Die Biegbarkeit des Schlauches ist derart, dass er bei der endoskopischen Verwendung der Vorrichtung durch die üblicherweise mittels des Endoskops auf den Schlauch einwirkenden Kräfte elastisch gebogen werden kann. Die Außenhülle begrenzt ein Lumen, vorzugsweise ein einziges zusammenhängendes Lumen, das sich entlang der Außenhülle vom proximalen Ende bis zum distalen Ende der Außenhülle erstreckt. Die Außenhülle ist sozusagen hohlzylindrisch.

Bei einem bevorzugten Ausführungsbeispiel ragt das Kopfteil rechtwinklig zur Längsachse betrachtet an keiner Stelle über eine virtuelle Zylindermantelfläche hinaus, wobei der Durchmesser der virtuellen Zylindermantelfläche dem maximalen Außendurchmesser der Außenhülle entspricht. Die virtuelle Zylindermantelfläche kann eine Verlängerung der Umfangsfläche der Außenhülle von ihrem distalen Ende oder einer Stelle mit maximalem Außendurchmesser in distale Richtung sein. Durch diese Dimensionierung können das Kopfteil und die Außenhülle besonders vorteilhaft durch den Arbeitskanal eines Endoskops in ein Körperlumen eines menschlichen oder tierischen Patienten eingebracht werden.

Alternativ dazu kann das Kopfteil teilweise oder vollständig aus einem elastisch verformbaren Material bestehen, so dass es zumindest in einem elastisch verformten Zustand nicht durch die virtuellen Zylindermantelfläche hinausragt und im elastisch nicht verformten Ausgangszustand einen Bestandteil oder mehrere Bestandteile aufweist, die durch die virtuelle Zylindermantelfläche hinausragen. Beispielsweise kann das Kopfteil wenigstens einen seitlich neben der Kopfteilöffnung angeordneten, in Querrichtung oder schräg zur Querrichtung von der Kopfteilöffnung weg ragenden Fußteil aufweisen. An dem wenigstens einen Fußteil kann die Auflagefläche vorhanden sein, die sich dadurch in Querrichtung vergrößern lässt. Der wenigstens eine Fußteil oder das gesamte Kopfteil kann elastisch verformbar sein. In seinem Ausgangszustand kann der wenigstens eine Fußteil durch die virtuelle Zylindermantelfläche hinausragen und aus diesem Ausgangszustand (beispielsweise manuell) nach innen zur Kopfteilöffnung hin und/oder über die Kopfteilöffnung und/oder in die Kopfteilöffnung hinein elastisch verformt werden.

In dem Lumen der Außenhülle sind vorzugsweise der Zufuhrkanal und/oder der Absaugkanal vorhanden. Bei einem Ausführungsbeispiel ist durch das Lumen ein Zufuhrschlauch geführt, der den Zufuhrkanal bildet und fluidisch mit dem Düsenrohr und/oder Düsenschlauch verbunden ist. Es ist insbesondere bevorzugt, wenn der Absaugkanal unmittelbar durch zumindest einen Bereich der Außenhülle begrenzt ist und somit der Absaugkanal zumindest durch einen Teil des Lumens gebildet ist, beispielsweise durch einen im Querschnitt durch die Außenhülle betrachtet ringförmigen Teil des Lumens.

Es ist vorteilhaft, wenn das Kopfteil um eine Drehachse drehbar gelagert ist. Die Drehachse kann der Längsachse entsprechen oder parallel versetzt zur Längsachse angeordnet sein. Das Kopfteil kann bei einer Ausführungsform durch die auf das Kopfteil von außen einwirkenden Kräfte sozusagen passiv bzw. ungesteuert drehbar gelagert sein. Bei diesem Ausführungsbeispiel ist keine die Drehlage des Kopfteils um die Drehachse beeinflussende Bedieneinrichtung an der Vorrichtung vorhanden. Alternativ hierzu kann eine Drehverbindungseinrichtung vorhanden sein, um die Drehlage des Kopfteils an einem proximal vorhandenen Bedienelement gezielt einstellen zu können.

Bei einem Ausführungsbeispiel, bei dem die Drehlage des Kopfteils steuerbar ist, ist ein Drehverbindungsglied vom Kopfteil zu einem Bedienelement, wobei sich das Drehverbindungsglied vorzugsweise durch das Lumen der Außenhülle erstreckt. Das Bedienelement kann beispielsweise an einem Handbedienteil der Vorrichtung angeordnet sein, ausgehend von dem sich die Außenhülle bis zum Kopfteil erstreckt. Das Drehverbindungsglied ist dazu eingerichtet, ein Drehmoment vom Bedienelement auf das Kopfteil zu übertragen, um dessen Drehlage um die Drehachse zu ändern bzw. einzustellen. Das Drehverbindungsglied kann beispielsweise mit seinem proximalen Ende drehfest mit dem Bedienelement und mit seinem distalen Ende drehfest mit dem Kopfteil verbunden sein.

Das Drehverbindungsglied, die Zufuhrleitung und die Außenhülle sind insbesondere quer zu ihrer Erstreckungsrichtung flexibel elastisch biegbar, bei von außen einwirkenden Kräften, die bei bestimmungsgemäßem Gebrauch (zum Beispiel in Verbindung mit einem Endoskop) auftreten.

Die Außenhülle und der Zufuhrschlauch sind vorzugsweise aus einem Kunststoff hergestellt, beispielsweise Silikon, Polyurethan, einem thermoplastischen Elastomer, insbesondere einem thermoplastischen Polyamide-Elastomer, die insbesondere derart ausgestaltet sind, dass die oben erwähnte elastische Biegbarkeit quer zu ihrer Erstreckungsrichtung gegeben ist.

Das Drehverbindungsglied kann beispielsweise eine oder mehrere Lagen von jeweils einem oder mehreren schraubenförmig gewundenen und/oder verflochtenen und/oder verdrillten Drähten aufweisen (z.B. wie es bei Stahlseilen bekannt ist), wodurch sich beispielsweise eine elastische Biegbarkeit quer zu seiner Erstreckungsrichtung realisieren lässt.

Das Drehverbindungsglied kann optional auch als laserstrukturiertes Rohr ausgeführt sein. Die Strukturierung ist insbesondere derart, dass das laserstrukturierte Rohr bei den in bestimmungsgemäßem Gebrauch auftretenden Kräften biegbar bzw. biegeweich ist. Das laserstrukturierte Rohr kann bei den in bestimmungsgemäßem Gebrauch auftretenden Kräften insbesondere derart torsionssteif ist, dass keine oder keine nennenswerte Torsion auftritt. Das Rohr kann optional ummantelt sein, um die fluidische Zuleitung (Wasserstrahl) zu übernehmen.

Das Drehverbindungsglied kann mit oder ohne einen sich entlang des Drehverbindungsgliedes erstreckenden Durchgangskanal ausgeführt sein. Wenn das Drehverbindungsglied einen Durchgangskanal aufweist, kann der Zufuhrkanal im Durchgangskanal des Drehverbindungsgliedes verlaufen, beispielsweise kann der Zufuhrschlauch durch den Durchgangskanal hindurchgeführt werden.

Es kann alternativ dazu auch vorteilhaft sein, das Drehverbindungsglied im Zufuhrkanal anzuordnen und beispielsweise durch den Zufuhrschlauch zu führen. Bei dieser Ausführungsform ist das Drehverbindungsglied vorzugsweise ohne Durchgangskanal ausgebildet und sozusagen ein im Wesentlichen solider Körper. Dabei können kleinere Zwischenräume im Drehverbindungsglied vorhanden sein, wenn dieses durch Verdrillen, Flechten oder anderweitiges Verbinden aus mehreren Drähten gebildet ist.

Zur Drehbarkeit des Kopfteils kann ein Drehlager zwischen der Außenhülle und dem Kopfteil und vorzugsweise einem Lagerfortsatz des Kopfteils gebildet sein. Beispielsweise kann der Lagerfortsatz des Kopfteils in das distale Ende der Außenhülle bzw. des Außenschlauchs hineinragen. Auf diese Weise kann zwischen dem Kopfteil und der Außenhülse ein Drehlager in Form eines Gleitlagers gebildet sein. Dadurch lässt sich eine einfache und kostengünstige Drehlagerung realisieren. Eine derartige Drehlagerung gestattet es, die Vorrichtung im Bereich des Kopfteils kompakt auszugestalten, so dass die Dimensionen radial zur Längsachse klein gehalten werden können. Die Vorrichtung ist in dieser Ausgestaltung sehr gut für den Einsatz in Kombination mit einem Endoskop geeignet.

An dem Lagerfortsatz des Kopfteils ist insbesondere zur fluidischen Verbindung des Innenraums mit dem Absaugkanal wenigstens ein Fluiddurchgang vorhanden. Mittels des Fluiddurchgangs ist der Lagerfortsatz für das Hindurchführen einer Fluidströmung eingerichtet. Der wenigstens eine Fluiddurchgang kann fluidisch verzweigt oder unverzweigt sein und beispielsweise abschnittsweise durch dazwischen angeordnete Stützelemente des Lagerfortsatzes fluidisch unterteilt sein.

Es ist bevorzugt, wenn das Düsenrohr und/oder der Düsenschlauch relativ zum Kopfteil unbeweglich angeordnet ist. Insbesondere ist das Düsenrohr und/oder der Düsenschlauch relativ zum Kopfteil nicht um die Längsachse drehbar oder drehbeweglich. Bei Ausführungsbeispielen, in denen das Kopfteil um die Drehachse drehbar ist, dreht sich das Düsenrohr bzw. der Düsenschlauch gemeinsam mit dem Kopfteil, so dass die Ausrichtung der Düsenöffnungen zur Kopfteilöffnung hin erhalten bleibt.

Die Vorrichtung kann vorteilhaft gemeinsam mit einem Endoskop verwendet werden. Hierzu kann das Endoskop beispielsweise einen Arbeitskanal aufweisen, durch den das Kopfteil und die Außenhülle der Vorrichtung hindurchgeführt werden können. Im Bereich des proximalen Endes können der Zufuhrkanal an eine Flüssigkeitsquelle und der Absaugkanal an eine Sammeleinrichtung angeschlossen werden, wobei die Sammeleinrichtung eine Saugeinheit oder Unterdruckeinheit aufweisen kann, um die Absaugströmung zu erzeugen.

Irgendein vorstehend beschriebenes Ausführungsbeispiel der Vorrichtung bzw. der Anordnung aus einem Endoskop mit einer solchen Vorrichtung kann wie folgt verwendet werden:

Das Kopfteil wird mit seiner Auflagefläche auf eine Gewebeoberfläche aufgelegt. Dadurch kann der Innenraum im Kopfteil zumindest im Wesentlichen gemeinsam mit der Gewebeoberfläche von der Umgebung des Kopfteils abgetrennt werden, um dort Gewebepartikel aufzufangen und anschließend abzuführen. Zum Abtragen von Gewebepartikeln von der Gewebeoberfläche oder einem oberflächennahen Bereich des Gewebes wird unter Druck stehende Flüssigkeit zum Düsenrohr bzw. Düsenschlauch geleitet. Dadurch entsteht an jeder Düsenöffnung des Düsenrohrs bzw. Düsenschlauchs ein Flüssigkeitsstrahl, der zunächst einen Teil des Innenraums durchquert und dann durch die Kopfteilöffnung auf das Gewebe auftrifft. Dadurch werden dort Gewebepartikel bzw. Zellen abgelöst, die sich dann im Innenraum des Kopfteils befinden. Die Gewebepartikel können dann über das Erzeugen einer Absaugströmung aus dem Innenraum und weiter über den Absaugkanal zu einer Partikelsammeleinrichtung gefördert werden.

Das Erzeugen der Flüssigkeitsstrahlen und das Erzeugen einer Absaugströmung kann gleichzeitig oder zumindest teilweise zeitlich überlappend oder zeitlich nacheinander durchgeführt werden.

Während des Erzeugens der Flüssigkeitsstrahlen und/oder während des Erzeugens der Absaugströmung kann das Kopfteil entlang der Gewebeoberfläche bewegt werden. Es ist auch möglich, die Bewegung des Kopfteils nur dann auszuführen, wenn keine Flüssigkeitsstrahlen erzeugt werden und/ oder wenn keine Absaugströmung erzeugt wird.

Vorteilhafte Ausgestaltungen der Erfindung ergeben sich aus den abhängigen Ansprüchen, der Beschreibung und der Zeichnung. Nachfolgend werden Ausführungsbeispiele der Erfindung basierend auf der beigefügten Zeichnung im Einzelnen erläutert. In der Zeichnung zeigen:
Figur 1 eine schematische Darstellung einer Anordnung aufweisend ein Endoskop mit einer erfindungsgemäßen Vorrichtung während einer beispielhaften Anwendung an einem Patienten,
Figur 2 eine schematische Prinzipdarstellung eines Ausführungsbeispiels einer erfindungsgemäßen Vorrichtung,
Figur 3 ein Ausführungsbeispiel eines Kopfteils, einer erfindungsgemäßen Vorrichtung in einer Ansicht rechtwinklig zu einer Kopfteilöffnung,
Figur 4 das Ausführungsbeispiel des Kopfteils aus Figur 3 in einem Längsschnitt entlang einer Längsachse durch das Kopfteil,
Figur 5 einen Querschnitt durch einen distalen Endabschnitt einer mit dem Kopfteil verbundenen Außenhülle, gemäß Schnittlinie A-A in Figur 4,
Figur 6 eine Darstellung eines Ausführungsbeispiels eines Handbedienteils der Vorrichtung gemäß Figur 2 in einem Längsschnitt,
Figur 7 ein Ausführungsbeispiel eines Kopfteils sowie eines mit dem Kopfteil verbundenen distalen Endbereich einer Außenhülle in einem Längsschnitt,
Figur 8 ein weiteres Ausführungsbeispiel eines Handbedienteils der Vorrichtung gemäß Figur 2 in einem Längsschnitt,
Figur 9 ein weiteres Ausführungsbeispiel eines Kopfteils sowie eines distalen Endbereichs einer Außenhülle der Vorrichtung und
Figur 10 einen Querschnitt durch das Kopfteil aus Figur 9 gemäß der Schnittlinie B-B in Figur 9.

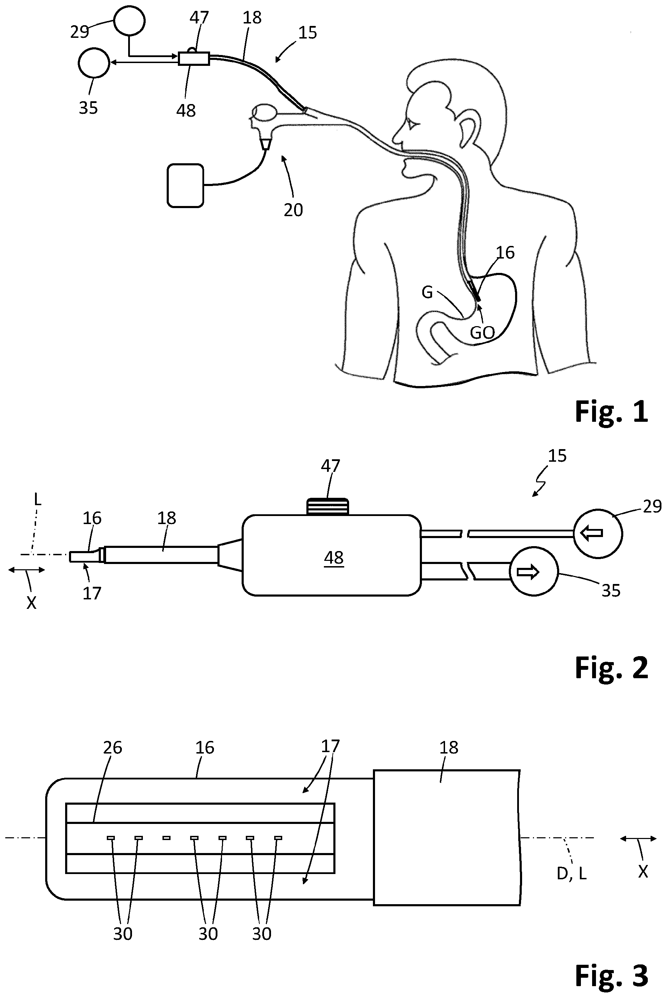

Die vorliegende Erfindung betrifft ein Verfahren und eine Vorrichtung 15, die dazu eingerichtet ist, Gewebepartikel P von einem Gewebe G, insbesondere von einer Gewebeoberfläche GO oder einem oberflächennahen Bereich des Gewebes G abzutragen und zu sammeln. Die Vorrichtung 15 ist schematisch in Prinzipdarstellungen in den Figuren 1 und 2 zu erkennen.

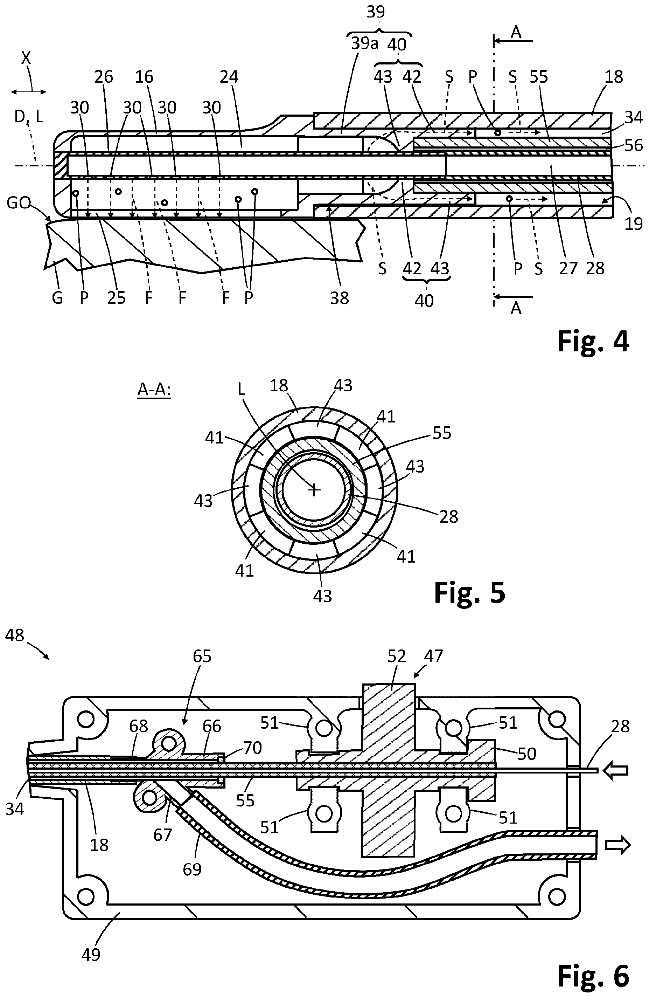

Die Vorrichtung 15 hat ein Kopfteil 16 mit einer Auflagefläche 17, die zur Auflage auf der Gewebeoberfläche GO eingerichtet ist. Das Kopfteil 16 ist an einem distalen Ende einer Außenhülle 18 angeordnet. Die Außenhülle 18 ist beispielsgemäß durch einen Schlauch gebildet. Durch die Außenhülle 18 erstreckt sich von einem proximalen Ende bis zu einem distalen Ende der Außenhülle 18 ein durchgängiges Lumen 19 (Figuren 4, 7 und 9). Die Außenhülle 18 ist quer zu ihrer Erstreckungsrichtung elastisch biegbar und derart flexibel, dass sie bei den üblicherweise auftretenden Kräften während eines bestimmungsgemäßen Gebrauchs der Vorrichtung 15 gebogen werden kann, insbesondere durch Kräfte, die von einem Endoskop auf die Außenhülle 18 ausgeübt werden, wenn die Außenhülle 18 sich durch einen Arbeitskanal des Endoskops 20 erstreckt (Figur 1).

Eine Anordnung bestehend aus dem Endoskop 20 und dem Instrument 15 ist in der beispielhaften Prinzipdarstellung in Figur 1 veranschaulicht. Mittels der Vorrichtung 15 können vorzugsweise in Kombination mit einem Endoskop 20 Gewebepartikel P von einem Gewebe G bzw. einer Gewebeoberfläche GO abgetragen und gesammelt werden. Das Gewebe G kann beispielsweise eine Schleimhaut sein, wie zum Beispiel die Magenschleimhaut.

Das Kopfteil 16 begrenzt einen Innenraum 24, der an einer Seite durch eine Kopfteilöffnung 25 nach außen offen und somit von außen zugänglich ist. Vorzugsweise ist das Kopfteil 16 abgesehen von der Kopfteilöffnung 25 ansonsten gegenüber der Umgebung des Kopfteils geschlossen (Figuren 4, 7, 9 und 10). Alternativ dazu kann auch wenigstens eine Belüftungsdurchbrechung (z.B. Durchgangsloch) vorhanden sein, die den Innenraum 24 fluidisch mit der Umgebung des Kopfteils 16 verbindet, um eine fluidische Einströmung aus der Umgebung in den Innenraum 24 zu ermöglichen.

In den Innenraum 24 ragt ein Düsenrohr 26 hinein, das sich entlang einer Längsachse L erstreckt. Das Düsenrohr 26 kann den Innenraum 24 in einer Längsrichtung X (parallel zur Längsachse L) vollständig durchsetzen und kann beispielsweise an beiden Enden mit dem Kopfteil 16 verbunden sein. Das Düsenrohr 26 ist insbesondere drehfest und vorzugsweise unbeweglich am Kopfteil 16 befestigt, beispielsweise formschlüssig und/oder kraftschlüssig und/oder stoffschlüssig und/oder mittels einer Klebeverbindung. An einer Stirnseite am distalen Ende ist das Düsenrohr 26 geschlossen. Am proximalen Ende ist das Düsenrohr 26 fluidisch mit einem Zufuhrkanal 27 verbunden. Als Zufuhrkanal 27 dient beim Ausführungsbeispiel ein Zufuhrschlauch 28. Der Zufuhrschlauch 28 ist analog zur Außenhülle 18 quer zu seiner Erstreckungsrichtung flexibel biegbar.

Bei allen Ausführungsbeispielen kann alternativ zum Düsenrohrs 26 auch ein Düsenschlauch oder eine Kombination aus Düsenrohr 26 auch Düsenschlauch verwendet werden. Bei den hier angegebenen Ausführungsbeispielen wird ein Düsenrohr 26 eingesetzt.

Die Außenhülle 18 und der Zufuhrschlauch 28 sind vorzugsweise aus einem Kunststoff hergestellt, beispielsweise Silikon, Polyurethan, einem thermoplastischen Elastomer, insbesondere einem thermoplastischen Polyamide-Elastomer.

Der Zufuhrkanal 27 ist fluidisch mit einer Flüssigkeitsquelle 29 verbindbar, über die unter Druck stehende Flüssigkeit durch den Zufuhrkanal 27 bzw. dem Zufuhrschlauch 28 in das Düsenrohr 26 geleitet werden kann.

Das Düsenrohr 26 hat mehrere Düsenöffnungen 30, wobei beispielsweise mindestens 3 oder mindestens 5 und vorzugsweise maximal 8 oder maximal 12 Düsenöffnungen vorhanden sein können. Die Anzahl der Düsenöffnungen ermöglicht einen guten Kompromiss zwischen einer möglichst flächigen Fluidabstrahlung bzw. Abtastung einerseits und einer Limitierung des erforderlichen Volumenstroms andererseits.

Die Düsenöffnungen 30 sind in einer Längsrichtung X parallel zur Längsachse L mit Abstand zueinander angeordnet. Der Abstand zwischen zwei unmittelbar benachbarten Düsenöffnungen beträgt beispielsweise 0,5 mm bis 1,0 mm. Die Dimension der Düsenöffnungen 30 in Längsrichtung X (Länge) ist vorzugsweise größer als die in einer Querrichtung Q (Breite). Die Düsenöffnungen 30 haben daher eine längliche Form, beispielsweise eine Schlitzform.

Die Längsrichtung X und die Querrichtung Q sind rechtwinkelig zueinander ausgerichtet.

Die Düsenöffnungen 30 sind beispielsgemäß in Längsrichtung X entlang einer Geraden und somit in einer einzigen Reihe angeordnet. Die Düsenöffnungen 30 sind auf der der Kopfteilöffnung 25 zugewandten Seite des Düsenrohres 26 angeordnet. Wenn dem Inneren des Düsenrohrs 26 über den Zufuhrkanal 27 unter Druck stehende Flüssigkeit zugeführt wird, tritt die Flüssigkeit an den Düsenöffnungen 30 aus, so dass sich an jeder Düsenöffnung 30 ein kompakter Fluidstrahl F bildet und in Richtung der Kopfteilöffnung 25 ausgestoßen wird (beispielhaft in Figur 4 gezeigt).

Die Fluidstrahlen F sind vorzugsweise parallel zueinander ausgerichtet. Beim Ausführungsbeispiel sind die Fluidstrahlen F in einer gemeinsamen Ebene angeordnet, in der sich auch die Längsachse L erstreckt. Durch die Kopfteilöffnung 25 hindurch können die Fluidstrahlen F auf die Gewebeoberfläche GO gerichtet werden, wenn das Kopfteil 16 mit der Auflagefläche 17 auf die Gewebeoberfläche GO des Gewebes G aufgelegt ist, wie es schematisch in Figur 4 dargestellt ist.

Beim Ausführungsbeispiel ist die Auflagefläche 17 unmittelbar benachbart zur Kopfteilöffnung 25 angeordnet. Die Auflagefläche 17 kann die Kopfteilöffnung 25 an mehreren Stellen und/oder auf mehreren Seiten begrenzen. Vorzugsweise erstreckt sich die Auflagefläche 17 kontinuierlich geschlossen um die Kopfteilöffnung 25 herum (Figur 3).

Der Innenraum 24 ist außerdem fluidisch mit einem Absaugkanal 34 verbunden. Bei einem Ausführungsbeispiel kann der Absaugkanal 34 durch die Außenhülle 18 begrenzt sein und im Lumen 19 der Außenhülle 18 vorhanden sein, beispielsweise in Form eines Ringkanals, der den Zuführschlauch 28 umgibt (Figuren 4, 7 und 9). An seinem proximalen Ende ist der Absaugkanal 34 fluidisch mit einer Partikelsammeleinrichtung 35 verbindbar (Figuren 1 und 2). Die Partikelsammeleinrichtung 35 kann eine Saugeinheit oder Unterdruckeinheit aufweisen, um eine Absaugströmung S vom Innenraum 24 durch den Absaugkanal 34 in die Partikelsammeleinrichtung 35 zu erzeugen (Figur 4). Mittels dieser Absaugströmung S können vom Gewebe G bzw. der Gewebeoberfläche GO abgelöste Gewebepartikel P bzw. Gewebezellen aus dem Innenraum 24 über den Absaugkanal 34 in die Partikelsammeleinrichtung 35 gefördert und dort gesammelt werden. In der Partikelsammeleinrichtung können ein oder mehrere Partikelsammelgefäße vorhanden sein, so dass die in einem Partikelsammelgefäß gesammelten Gewebepartikel P einer bestimmten Stelle des Gewebes G bzw. der Gewebeoberfläche GO zugeordnet werden können, von der die Gewebepartikel P abgetragen und gesammelt worden sind.

Der Begriff "proximal" definiert eine Richtung oder Position in Bezug auf die Vorrichtung 15 oder ein Teil davon zur Fluidquelle 29 und/oder zur Partikelsammeleinrichtung 35 hin. Der Begriff "distal" definiert eine Richtung oder Position in Bezug auf die Vorrichtung 15 oder ein Teil davon von der Fluidquelle 29 und/oder der Partikelsammeleinrichtung 35 weg. Die Begriffe "proximal" und "distal" werden nicht nur in Bezug auf die gesamte Vorrichtung 15, sondern auch bei der Beschreibung individueller Bestandteile davon in Bezug auf den jeweils beschriebenen Bestandteil verwendet.

Die insoweit beschriebene Vorrichtung 15 kann wie folgt zur Entnahme von Gewebepartikeln P eingesetzt werden:

Das Kopfteil 16 wird mit der Auflagefläche 17 auf eine Gewebeoberfläche GO eines Gewebes G aufgelegt, von der Gewebepartikel P abgetragen werden sollen. Durch Zufuhr von unter Druck stehender Flüssigkeit F von der Flüssigkeitsquelle 29 über den Zufuhrkanal 27 in das Düsenrohr 26 entsteht an jeder Düsenöffnung 30 ein Flüssigkeitsstrahl F, der an der Düsenöffnung 30 austritt, einen Teil des Innenraums 24 durchquert und durch die Kopfteilöffnung 25 auf die Gewebeoberfläche GO auftrifft. Auf der Gewebeoberfläche GO werden schonend und insbesondere unblutig Gewebepartikel P bzw. Gewebezellen abgelöst bzw. abgetragen und befinden sich dann zunächst in dem durch das Kopfteil 16 begrenzten Innenraum 24. Die losen Gewebepartikel P werden beim Erzeugen einer Absaugströmung S mittels einer geeigneten Saugeinheit oder Unterdruckeinheit aus dem Innenraum 24 in den Absaugkanal 34 und vom Absaugkanal 34 in die Partikelsammeleinrichtung 35 gefördert. Die losen Gewebepartikel P können mit der Flüssigkeit sozusagen eine Suspension bilden. Dort können die Gewebepartikel P in einem oder optional mehreren Partikelsammelbehältern gesammelt werden, so dass sie beispielsweise für eine weitere Gewebeanalyse zur Verfügung stehen.

Zusätzlich oder alternativ kann mittels der Vorrichtung 15 auch eine Gewebereinigung oder eine andere Gewebebeeinflussung der Gewebeoberfläche GO bzw. von oberflächennahen Schichten des Gewebes G durchgeführt werden.

Bei den bevorzugten Ausführungsbeispielen ist das Kopfteil 16 um eine Drehachse D drehbar an der Außenhülle 18 gelagert. Die Drehachse D entspricht vorzugsweise der Längsachse L. Hierzu kann zwischen dem Kopfteil und der Außenhülle 18 ein Drehlager 38 gebildet sein, das insbesondere als Gleitlager ausgeführt ist.

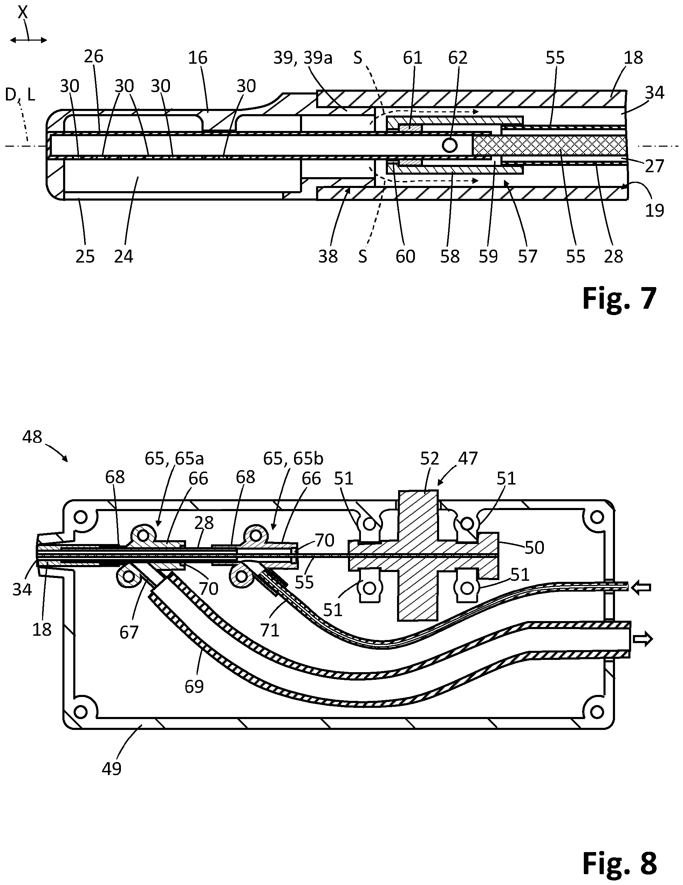

Bei den Ausführungsbeispielen gemäß der Figuren 4 und 7 weist das Kopfteil 16 einen Lagerfortsatz 39 auf, dessen Umfangsfläche die Drehachse D und beispielsgemäß die Längsachse L koaxial umgibt. Der Lagerfortsatz 39 ist in das distale Ende der Außenhülle 18 eingesteckt und liegt mit seiner Umfangsfläche gleitend drehbeweglich an der Innenfläche der Außenhülle 18 an. Hierzu wird eine geeignete Materialpaarung und Dimensionierung gewählt. Durch die Anlage der Außenhülle 18 am Lagerfortsatz 39 kann der ringförmige Absaugkanal 34 zur Umgebung des Kopfteils 16 hin zumindest im Wesentlichen fluiddicht abgeschlossen sein, was allerdings nicht zwingend erforderlich ist. Wenn an dieser Stelle beim Auftreten einer Absaugströmung S Gas aus der das Kopfteil 16 umgebenden Atmosphäre angesaugt wird, ist dies unkritisch und kann vorteilhaft sein, um das Festsaugen des Kopfteils 16 an der Gewebeoberfläche GO zu vermeiden und die Verschiebbarkeit des Kopfteils 16 entlang der Gewebefläche GO während der Erzeugung einer Absaugströmung S erleichtern. Wie bereits erwähnt kann zur Vermeidung eines derartigen Festsaugens auch wenigstens eine Belüftungsdurchbrechung am Kopfteil 16 vorhanden sein, das den Innenraum 24 fluidisch mit der Umgebung verbindet.

Bei dem in den Figuren 4 und 7 veranschaulichten Ausführungsbeispielen hat der Lagerfortsatz 39 einen hohlzylindrischen Teil 39a und kann ausschließlich durch diesen hohlzylindrischen Teil 39a gebildet sein, wie beim Ausführungsbeispiel nach Figur 7. Im Unterschied dazu kann der Lagerfortsatz 39 beim Ausführungsbeispiel nach Figur 4 mehrere Stützelemente 40 haben, die in Umfangsrichtung um die Längsachse L verteilt angeordnet sind. Dadurch wird zwischen zwei in Umfangsrichtung um die Längsachse L unmittelbar benachbarten Stützelementen 40 jeweils ein Fluiddurchgang 41 gebildet, um eine fluidische Verbindung zwischen dem Absaugkanal 34 und dem Innenraum 24 herzustellen. Die Stützelemente 40 schließen sich in Längsrichtung X an den hohlzylindrischen Teil 39a des Lagerfortsatzes 39 an. Im Übergangsbereich zum hohlzylindrischen Teil 39a des Lagerfortsatzes 39 hat jedes Stützelement 40 beispielsgemäß einen radial nach innen zur Längsachse L hin wegragenden Radialvorsprung 42 (Figur 4). An den Radialvorsprung schließt sich ein sich in Längsrichtung X erstreckender Längssteg 43 an. Die von der Längsachse L abgewandte Außenfläche jedes Längssteges 43 erstreckt sich in Längsrichtung X in Verlängerung der Umfangsfläche des hohlzylindrischen Teils 39a des Lagerfortsatzes 39.

Bei den Ausführungsbeispielen gemäß der Figuren 4 und 7 ist das Kopfteil 16 gemeinsam mit dem daran angeordneten Düsenrohr 26 mittels eines Bedienelements 47 an einem Handbedienteil 48 der Vorrichtung 15 drehbar. Über das Bedienelement 47 kann eine Drehbewegung bzw. ein Drehmoment auf das Kopfteil 16 ausgeübt werden, um dessen Drehlage um die Drehachse D bzw. die Längsachse L zu ändern und einzustellen. Das Handbedienteil 48 mit dem Bedienelement 47 ist in den Figuren 1, 2, 6 und 8 zu erkennen. Es hat ein Gehäuse 49, an dem das Bedienelement 47 drehbar gelagert ist. Dazu weist das Bedienelement 47 einen Drehzapfen 50 auf, der an zwei beabstandeten Stellen drehend an Drehlagerteilen 51 des Gehäuses 49 abgestützt ist. Beispielsgemäß sind die Drehlagerteile 51 im Inneren des Gehäuses 49 angeordnet. Am Drehzapfen 50 ist eine Drehscheibe oder ein Drehring 52 des Bedienelements 47 drehfest angeordnet und ragt zumindest an einer Stelle durch eine Gehäuseöffnung aus dem Gehäuse 49 heraus. An dieser Stelle ist das Bedienelement 47 bzw. der Drehring 52 zugänglich, so dass das Bedienelement 47 um die Achse des Drehzapfens 50 gedreht werden kann. Der Drehring 52 ist beim Ausführungsbeispiel durch eine geschlossene Scheibe gebildet, kann aber auch über speichenartige Verbindungen mit dem Drehzapfen 50 verbunden sein.

Zur Übertragung der Drehbewegung des Bedienelements 47 zum Kopfteil 16 ist eine sich durch das Lumen 19 der Außenhülle 18 erstreckende Drehverbindung vorhanden, die beispielsgemäß durch ein Drehverbindungsglied 55 gebildet ist. Das Drehverbindungsglied 55 ist quer zu seiner Erstreckungsrichtung elastisch biegbar ausgebildet. Es kann beispielsweise einen oder mehrere schraubenförmig gewundene Drähte aufweisen. Mehrere solcher Drähte können miteinander verwoben, verdrillt oder auf andere geeignete Weise verbunden sein. Das Drehverbindungsglied 55 kann mehrere koaxiale Lagen aus jeweils einem oder mehreren schraubenförmig gewundenen Drähten aufweisen.

Das Drehverbindungsglied 55 ist an seinem proximalen Ende drehfest mit dem Bedienelement 47 verbunden und beispielsgemäß mit dem Drehzapfen 50. Bei einer Drehung des Bedienelements 47 wird das Drehverbindungsglied 55 um seine Mittelachse gedreht und überträgt diese Drehbewegung auf das Kopfteil 16. Dazu ist das distale Ende des Drehverbindungsgliedes 55 drehfest mit dem Kopfteil 16 verbunden.

Bei dem in Figur 4 dargestellten Ausführungsbeispiel ist das distale Ende des Drehverbindungsgliedes 55 unmittelbar drehfest mit dem Kopfteil 16 verbunden, beispielsgemäß dem Lagerfortsatz 39. Hierzu übergreifen beispielsgemäß die Stützelemente 40 des Lagerfortsatzes 39 den distalen Abschnitt des Drehverbindungsgliedes 55. Dabei können sowohl die radial Vorsprünge 42, als auch die Längsstege 43 der Stützelemente 40 am Drehverbindungsglied 55 anliegen, wie es schematisch in Figur 4 gezeigt ist.

In Abwandlung hierzu kann das Drehverbindungsglied 55 auch unmittelbar drehfest am Düsenrohr 26 befestigt sein (Figur 7) beispielsweise kann das distale Ende des Drehverbindungsgliedes 55 in das proximale Ende des Düsenrohres 26 hineinragen und dort kraftschlüssig und/oder stoffschlüssig und/oder formschlüssig und/oder mittels einer Haftverbindung befestigt sein.

Bei dem in Figur 7 dargestellten Ausführungsbeispiel hat das Drehverbindungsglied 55 eine rohrförmige oder schlauchförmige Gestalt mit einem, das Drehverbindungsglied 55 vom proximalen Ende bis zum distalen Ende durchsetzenden Durchgangskanal 56. Durch diesen Durchgangskanal 56 kann sich der Zufuhrkanal 27 erstrecken. Beispielsweise kann der Zufuhrschlauch 28 durch den Durchgangskanal 56 geführt werden, wie es in Figur 4 zu erkennen ist.

Bei dem in Figur 4 dargestellten Ausführungsbeispiel sind der Zufuhrschlauch 28 mit dem Zufuhrkanal 27, das Drehverbindungsglied 55 und die Außenhülle 18 koaxial zueinander angeordnet. Zwischen dem Drehverbindungsglied 55 und der Außenhülle 18 ist der Absaugkanal 34 vorhanden, der im distalen Bereich der Außenhülle durch die Längsstege 43 der Stützelemente 40 in die Fluiddurchgänge 41 unterteilt wird. Jeder Fluiddurchgang 41 mündet benachbart zum Düsenrohr 26 in den Innenraum 24. Somit können mehrere Kanalmündungen in Umfangsrichtung um die Längsachse L verteilt um das Düsenrohr 26 angeordnet sein.

Im Unterschied zur Ausführungsform nach Figur 4 ist das Drehverbindungsglied 55 beim Ausführungsbeispiel nach Figur 7 ohne einen Durchgangskanal ausgebildet. Bei dieser Ausführung ist das Drehverbindungsglied 55 im distalen Endbereich der Außenhülle 18 zentral in Verlängerung der Längsachse L mit dem Düsenrohr 26 verbunden und somit zentral in der Außenhülle 18 bzw. im Lumen 19 angeordnet. Koaxial um das Drehverbindungsglied 55 ist der Zufuhrschlauch 28 angeordnet. Bei diesem Ausführungsbeispiel ist der Zufuhrkanal 27 ringförmig um das Drehverbindungsglied 55 im Zufuhrschlauch 28 gebildet.

Zur fluidischen Verbindung mit dem Düsenrohr 26 ist beispielsgemäß eine Kopplungseinrichtung 57 vorhanden, die eine hohlzylindrische Kopplungshülse 58 aufweist. Die Kopplungshülse 58 ist fluiddicht mit dem distalen Ende des Zufuhrschlauchs 28 verbunden. Beispielsweise kann der Zufuhrschlauch 28 mit seinem distalen Ende in die Kopplungshülse 58 eingesteckt und dort verklebt, verschweißt oder anderweitig fluiddicht befestigt werden. Die Kopplungshülse 58 umgibt einen Fluidraum 59, in den das proximale Ende des Düsenrohres 26, das mit dem Drehverbindungsglied 55 verbunden ist, durch eine stirnseitige Öffnung 60 hineinragt.

Im Fluidraum 59 ist benachbart zur stirnseitigen Öffnung 60 eine Ringdichtung 61 angeordnet. Die Ringdichtung 61 liegt außen am Düsenrohr 26 an. Die Ringdichtung 61 liegt außerdem an einem Wandabschnitt der Kopplungshülse 58 benachbart zur stirnseitigen Öffnung 60 an. Auf diese Weise dichtet die Ringdichtung 61 den Fluidraum 59 gegenüber der Außenseite des Düsenrohres 26 und gegenüber der stirnseitigen Öffnung 60 ab. In dem in den Fluidraum 59 hineinragenden Abschnitt hat das Düsenrohr 26 eine oder mehrere Einlassöffnungen 62. Durch diese Einlassöffnungen kann eine über den Zufuhrkanal 27 in den Fluidraum 59 zugeführte Flüssigkeit in das Innere des Düsenrohres 26 gelangen und dann über die Düsenöffnungen 30 zur Bildung der Flüssigkeitsstrahlen F ausgestoßen werden.

Wenn das Bedienelement 47 gedreht wird, wird die Drehung auf das Drehverbindungsglied 55 übertragen, die die Drehung wiederum auf das Kopfteil 16 überträgt, entweder unmittelbar (Figur 4) oder mittelbar über das Düsenrohr 26 (Figur 7).

Beim Ausführungsbeispiel nach Figur 4 liegt der Zufuhrschlauch 28 ohne drehfeste Verbindung im Durchgangskanal 56 des Drehverbindungsgliedes 55. Wegen der Verbindung des Zufuhrschlauches 28 mit dem Düsenrohr 26 wird das distale Ende des Zufuhrschlauches 28 mitgedreht, wobei das proximale Ende des Zufuhrschlauches 28 beim bevorzugten Ausführungsbeispiel nicht gedreht wird. Wegen der Länge des Zufuhrschlauches 28 vom proximalen Ende zum distalen Ende kann die dabei entstehende Torsion des Zufuhrschlauches 28 toleriert werden. Die durch die Torsion des Zufuhrschlauches 28 bei einer einzigen vollständigen Umdrehung des Kopfteils 16 ausgehend von einer nicht tordierten Ruhelage des Zufuhrschlauches 28 sind gering und somit unkritisch werden.

Beim Ausführungsbeispiel nach Figur 7 ist über die Kopplungseinrichtung 57 das Düsenrohr 26 drehbar gelagert und kann sich relativ zur Ringdichtung 61 der Kopplungseinrichtung 57 drehen. Somit bleibt der Zufuhrschlauch 28 unabhängig von der Drehlage des Kopfteils 16 in einer nicht tordierten bzw. nicht gedrehten Stellung.

Die Vorrichtung 15 kann in einem Bereich zwischen dem proximalen Ende der Außenhülle 18 einerseits und der Flüssigkeitsquelle 29 bzw. der Partikelsammeleinrichtung 35 andererseits wenigstens ein Fluidkopplungselement 65 aufweisen. Jedes Fluidkopplungselement 65 ist dazu eingerichtet zwei innerhalb des Lumens 19 der Außenhülse 18 koaxial angeordnete Komponenten der Vorrichtung 15 (z.B. Zufuhrkanal 27 bzw. Zufuhrschlauch 28, Absaugkanal 34, Drehverbindungsglied 55) in eine nicht koaxiale Anordnung zu überführen und dabei die Fluiddichtheit eines betreffenden Kanals (z.B. Zufuhrkanal 27, Absaugkanal 34) aufrechtzuerhalten.

Wie bereits erläutert, ist die Flüssigkeitsquelle 29 und die Partikelsammeleinrichtung 35 (einschließlich einer der Saugeinheit bzw. Unterdruckeinheit) über das Gehäuse 49 des Handbedienteils 48 an den Zufuhrkanal 27 bzw. den Absaugkanal 34 angeschlossen. Mittels des wenigstens einen Fluidkopplungselements 65 können diese fluidische Verbindungen hergestellt werden, wie es anhand der Figuren 6 und 8 gezeigt ist. Das wenigstens eine Fluidkopplungselement 65 kann im Inneren des Gehäuses 49 angeordnet sein.

Zum Anschließen des Zufuhrschlauches 28, der innerhalb des Durchgangskanals 56 des Drehverbindungsgliedes 55 angeordnet ist (Figuren 4 und 6) kann der Zufuhrschlauch 28 aus dem proximalen Ende des Drehverbindungsgliedes 55 herausgeführt und durch eine geeignete Fluidverbindung mit der Flüssigkeitsquelle 29 verbunden werden. Hierzu kann beispielsweise der Drehzapfen 50 ein sich entlang seiner Längsachse erstreckendes Durchgangsloch aufweisen, durch das sich der Zufuhrschlauch 28 hindurch erstreckt. Innerhalb des Durchgangsloches kann das Drehverbindungsglied 55 drehfest mit dem Drehzapfen 50 verbunden sein (Figur 6).

Bei dem in Figur 6 dargestellten Ausführungsbeispiel ist ein einziges Fluidkopplungselement 65 ausreichend. Das Fluidkopplungselement 65 hat einen ersten Eingangskanal 66, einen zweiten Eingangskanal 67 und einen Ausgangskanal 68. Das Drehverbindungsglied 55 sowie die darin angeordnete Zufuhrleitung 28 erstrecken sich in den ersten Eingangskanal 66 hinein und aus dem Ausgangskanal 68 heraus. Mit dem Ausgangskanal 68 ist die Außenhülle 18 fluiddicht verbunden. Im Anschluss an den Ausgangskanal 68 sind daher der Zufuhrschlauch 28, das Drehverbindungsglied 55 und die Außenhülle 18 koaxial zueinander angeordnet.

Der zwischen der Außenhülle 18 und dem Drehverbindungsglied 55 vorhandene ringförmige Absaugkanal 34 ist im Fluidkopplungselement 65 fluidisch mit dem zweiten Eingangskanal 67 verbunden. Der zweite Eingangskanal 67 des Fluidkopplungselementes 65 ist fluidisch mit einem Absaugschlauch 69 verbunden, der aus dem Gehäuse 49 herausgeführt ist und mit der Partikelsammeleinrichtung 35 verbunden werden kann. Um den Absaugkanal 34 gegenüber dem Innenraum des Gehäuses 49 abzudichten, ist eine Fluiddichtung 70 am ersten Eingangskanal 66 angeordnet, die außen am Drehverbindungsglied 55 fluiddicht anliegt. Das Drehverbindungsglied 55 ist relativ zur Fluiddichtung 70 drehbar. Die Fluiddichtung 70 kann beispielsweise ein O-Ring sein.

Das in Figur 8 dargestellte Ausführungsbeispiel des Handbedienelements 48 weist zur Fluidverbindung des Absaugschlauches 69 mit dem Absaugkanal 34 dasselbe Fluidkopplungselement 65 auf, wie es vorstehend im Zusammenhang mit Figur 6 erläutert wurde.

Bei einer Vorrichtung 15, bei der das Drehverbindungsglied 55 innerhalb des Zufuhrschlauches 28 angeordnet ist (Figur 7) ist ein zusätzliches Fluidkopplungselement 65 im Gehäuse 49 vorhanden, wie es in Figur 8 gezeigt ist. Zur Unterscheidung ist in Figur 8 das auch beim Ausführungsbeispiel nach Figur 6 vorhandene Fluidverbindungselement 65 als erstes Fluidkopplungselement 65a bezeichnet, während das zusätzliche Fluidverbindungselement 65 als zweites Fluidkopplungselement 65b bezeichnet ist.

Das zweite Fluidkopplungselement 65b ist proximal zum ersten Fluidkopplungselement 65a angeordnet. Das zweite Fluidkopplungselement 65b hat prinzipiell denselben oben beschriebenen Aufbau wie das erste Fluidkopplungselement 65a und weist einen ersten Eingangskanal 66 mit einer Fluiddichtung 70, einen zweiten Eingangskanal 67 und einen Ausgangskanal 68 auf. Durch den ersten Eingangskanal 66 ist fluiddicht das Drehverbindungsglied 55 geführt und tritt durch den Ausgangskanal 68 aus. Mit dem Ausgangskanal 68 ist fluiddicht die Zufuhrleitung 28 verbunden, in die das Drehverbindungsglied 55 eingeführt ist, so dass die Zufuhrleitung 28 im Anschluss an den Ausgangskanal 68 das Drehverbindungsglied 55 im Zufuhrkanal 27 aufnimmt. Diese koaxiale Anordnung kann dann in den ersten Eingangskanal 66 des ersten Fluidkopplungselement 65a geführt werden.

Über den zweiten Eingangskanal 67 des zweiten Fluidkopplungselements 65b ist der Zufuhrkanal 27 im Zufuhrschlauch 28 fluidisch mit einer Fluidleitung 71 verbunden. Die Fluidleitung 71 ist vom zweiten Eingangskanal 67 ausgehend aus dem Gehäuse 49 herausgeführt und kann durch geeignete Verbindungsmittel mit der Flüssigkeitsquelle 29 fluidisch verbunden werden.

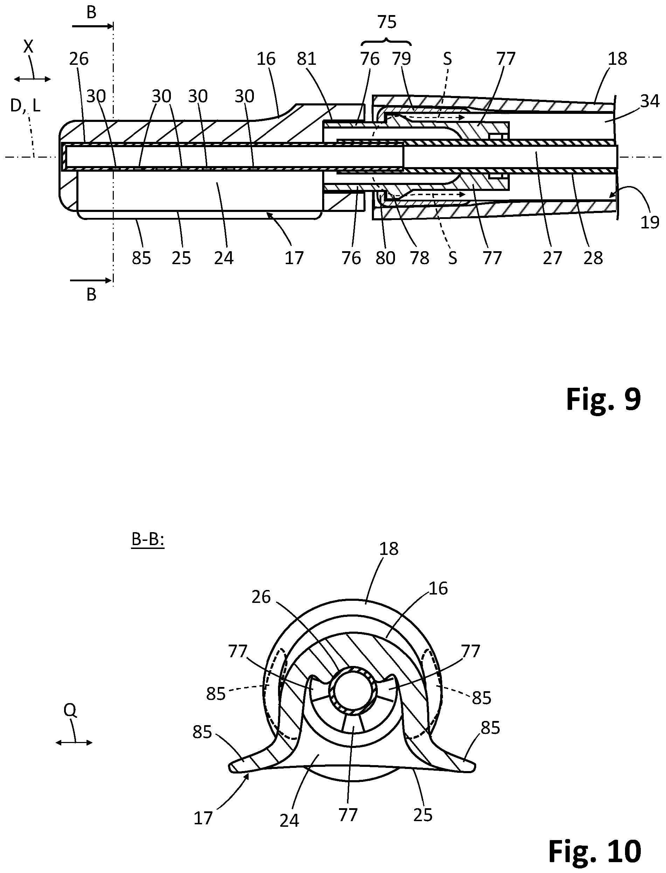

Bei den bisher erläuterten Ausführungsbeispielen der Vorrichtung 15 ist das Kopfteil 16 in seiner Drehlage um die Drehachse D bzw. die Längsachse L aktiv steuerbar oder einstellbar. In Abwandlung hierzu kann das Kopfteil 16 drehbar am distalen Ende der Außenhülle 18 gelagert sein, ohne eine Drehverbindung zum proximalen Ende, so dass das durch das Lumen 19 der Außenhülle 18 geführte Drehverbindungsglied 55 entfallen kann. Die Drehlage des Kopfteils 16 richtet sich dann nach den von außen auf das Kopfteil 16 einwirkenden Kräften, beispielsweise bei der Bewegung des Kopfteils 16 entlang einer Gewebeoberfläche GO oder durch Kräfte, die beim Erzeugen der Absaugströmung S und/oder der Fluidstrahlen F entstehen. Ein solches Ausführungsbeispiel ist in den Figuren 9 und 10 schematisch dargestellt.

Das Kopfteil 16 kann bei allen Ausführungsbeispielen mehrteilig sein und/oder in verschiedenen Bereichen sich unterscheidende Materialen aufweisen. Beispielsweise kann der Lagerfortsatz 39 aus einem anderen Material bestehen oder ein anderes Material enthalten als der verbleibende Bestandteil des Kopfteils 16.

Bei dem in den Figuren 9 und 10 veranschaulichten Ausführungsbeispiel hat das Kopfteil 16 keinen Lagerfortsatz 39. Das Kopfteil 16 ist mittelbar über eine Verbindungseinrichtung 75 mit dem distalen Ende der Außenhülle 18 verbunden. Die Verbindungseinrichtung 75 hat eine Verbindungshülse 76, die ähnlich ausgebildet sein kann, wie der Lagerfortsatz 39 beim Kopfteil 16 gemäß der Figuren 4 und 5. Die Verbindungshülse 76 kann insbesondere einen hohlzylindrischen Teil und sich daran in Längsrichtung X anschließende Verbindungselemente 77 aufweisen, die in Umfangsrichtung um die Längsachse L mit Abstand zueinander angeordnet sind, analog zu den Stützelementen 40, so dass dazwischen Fluiddurchgänge für die Absaugströmung S gebildet sind. Im Übergangsbereich zwischen dem hohlzylindrischen Teil und den Verbindungselementen 77 hat die Verbindungshülse 76 einen oder mehrere von der Längsachse L nach außen wegragende Haltevorsprünge 78. Beispielsweise kann ein einziger ringförmiger Haltevorsprung 78 vorhanden sein, oder es können mehrere in Umfangsrichtung um die Längsachse L mit Abstand zueinander angeordnete Haltevorsprünge 78 an der Verbindungshülse 76 vorhanden sein.

Zur Verbindung mit dem distalen Ende weist die Verbindungseinrichtung 75 außerdem eine Außenhülse 79 auf. Die Außenhülse 79 begrenzt einen Innenraum, in den die Verbindungshülse 76 einschließlich des wenigstens einen Haltevorsprungs 68 von der dem Kopfteil 16 abgewandten Seite eingeführt werden kann. An der dem Kopfteil 16 zugewandten Seite hat die Außenhülse 79 wenigstens einen Anschlag 80, der beispielsweise von einem Ringflansch oder einer Ringschulter gebildet sein kann. Alternativ können in Umfangsrichtung um die Längsachse L auch mehrere zur Längsachse L hinragende Anschläge 80 vorhanden sein. Die Anschläge 80 begrenzen eine Öffnung, durch die der hohlzylindrische Teil der Verbindungshülse 76 hindurchragen kann. Der wenigstens eine Haltevorsprung 78 liegt an dem wenigstens einen Anschlag 80 an. In Umfangsrichtung um die Längsachse L sind die Dimensionen des wenigstens einen Haltevorsprungs 78 und des wenigstens einen Anschlags 80 derart gewählt, dass in jeder Drehstellung um die Längsachse L ein Herausziehen der Verbindungshülse 76 aus der Außenhülse 79 vermieden ist. Dies kann beispielsweise dadurch erreicht werden, dass zumindest der Anschlag 80 oder der Haltevorsprung 78 ringförmig ausgeführt ist.

Die Außenhülse 79 ist mit dem distalen Ende der Außenhülle 18 verbunden, beispielsweise kraftschlüssig und/oder stoffschlüssig und/oder durch eine Haftverbindung. Die Verbindungshülse 76 ist drehbar in der Außenhülse 79 angeordnet. Die Verbindungshülse 76 ist drehfest mit dem Kopfteil 16 verbunden und kann beispielsweise drehfest in einer Aussparung 81 des Kopfteils 16 befestigt sein. Durch die Verbindungseinrichtung 75 können die Reibungskräfte zur Drehung des Kopfteils 16 um die Drehachse D beziehungsweise Längsachse L verringert werden, indem für die Verbindungshülse 76 und die Außenhülse 79 eine geeignete Materialpaarung gewählt wird. Die Verbindungshülse 76 und die Außenhülse 79 können beispielsweise aus Metall, einer metallischen Legierung, Keramik oder ähnlichem gebildet sein. Wegen der kurzen Länge in Längsrichtung X muss die Verbindungseinrichtung 75 quer zur Längsrichtung X nicht elastisch verformbar sein.

In Figur 10 ist beispielhaft ein Querschnitt durch das Kopfteil 16 gemäß der Schnittlinie B-B in Figur 9 gezeigt. Die Ausgestaltung dieses Kopfteils 16 kann bei sämtlichen Ausführungsbeispielen eingesetzt werden. Sie eignet sich insbesondere für ein Kopfteil 16, das nicht gesteuert über das Bedienelement 47 drehbar ist und lediglich (sozusagen passiv) drehbar am distalen Ende der Außenhülle 18 gelagert ist.

Das Kopfteil 16 hat bei der in Figur 10 dargestellten Ausführungsform auf zwei sich in einer Querrichtung Q gegenüberliegenden Seiten benachbart zur Kopfteilöffnung 25, jeweils einen von der Kopfteilöffnung 25 rechtwinklig oder schräg nach außen wegragenden Fußteil 85. Über diesen Fußteil 85 kann eine seitlich bzw. in Querrichtung breitere und somit vergrößerte Auflagefläche 17 gebildet werden.

Die Fußteile 85 und optional auch zumindest ein sich an die Fußteile 85 anschließender Abschnitt des Kopfteils 16 oder auch das gesamte Kopfteil 16 können bei dieser Ausführungsform aus einem elastisch verformbaren Material hergestellt sein, wobei die Materialien in Frage kommen, aus denen auch die Außenhülle 18 und/oder der Zufuhrschlauch 28 gebildet sind. Dadurch können die Fußteile aus einer nicht elastisch verformten Ausgangsstellung (durchgezogene Linie in Figur 10) in eine elastisch verformte Stellung (gestrichelte Linie in Figur 10) gebracht werden. In der elastisch verformten Stellung können sich die Fußteile über und/oder in die Kopfteilöffnung 25 erstrecken. Diese Stellung können die Fußteile 85 beispielsweise einnehmen, um den Kopfteil 16 durch den Arbeitskanal des Endoskops 20 zu bewegen. Außerhalb des Arbeitskanals und ohne äußere Einwirkung nehmen die Fußteile 85 (wieder) die nicht elastisch verformten Ausgangsstellung ein.

In Abwandlung zu den veranschaulichten Ausführungsbeispielen können bei sämtlichen Ausgestaltungen des Kopfteils 16 Öffnungen den Innenraum 24 mit der Umgebung des Kopfteils fluidisch verbinden, um beim Erzeugen der Ansaugströmung S ein zu starkes Haften des Kopfteils an der Gewebeoberfläche GO zu vermeiden.

Die Erfindung betrifft eine Vorrichtung 15 zum Abtragen von Gewebepartikeln P eines Gewebes G von der Gewebeoberfläche GO oder einem oberflächennahen Gewebebereich, insbesondere ohne Blutungen zu verursachen. Dadurch können Gewebepartikel P bzw. Zellen gesammelt werden, beispielsweise für eine spätere Gewebeanalyse. Die Vorrichtung 15 hat hierzu ein Kopfteil 16, das einen Innenraum 24 begrenzt, in den ein Düsenrohr 26 hineinragt. Das Düsenrohr hat mehrere benachbart zueinander angeordnete Düsenöffnungen 30, die zu einer Kopfteilöffnung 25 des Kopfteils 16 hin ausgerichtet sind. Benachbart zur Kopfteilöffnung 25 hat das Kopfteil 16 eine Auflagefläche 17, die zur Auflage auf der Gewebeoberfläche GO eingerichtet ist. Über einen Zufuhrkanal 27 kann unter Druck stehende Flüssigkeit in das Düsenrohr 30 eingeleitet und unter Bildung von kompakten Fluidstrahlen aus den Düsenöffnungen 30 ausgestoßen werden, um beim Auftreffen auf der Gewebeoberfläche GO Gewebepartikel P abzulösen, die sich dann im Innenraum 24 des Kopfteils 16 befinden. Der Innenraum 24 ist fluidisch mit einem Absaugkanal 34 verbunden. Über den Absaugkanal kann eine Absaugströmung S Gewebepartikel aus dem Innenraum 24 absaugen und in einer Partikelsammeleinrichtung 35 sammeln, die fluidisch mit dem Absaugkanal 34 verbindbar ist. Mittels einer solchen Vorrichtung 15 kann schonend eine Gewebeprobe in Form von Gewebepartikeln P entnommen werden. Die Vorrichtung 15 kann in Kombination mit einem Endoskop 20 eingesetzt werden.

### Bezugszeichenliste:

- 15: Vorrichtung
- 16: Kopfteil
- 17: Auflagefläche
- 18: Außenhülle
- 19: Lumen der Außenhülle
- 20: Endoskop

- 24: Innenraum
- 25: Kopfteilöffnung
- 26: Düsenrohr
- 27: Zufuhrkanal
- 28: Zufuhrschlauch
- 29: Flüssigkeitsquelle
- 30: Düsenöffnung

- 34: Absaugkanal
- 35: Partikelsammeleinrichtung

- 38: Drehlager
- 39: Lagerfortsatz
- 39a: hohlzylindrischer Teil des Lagerfortsatzes
- 40: Stützelement
- 41: Fluiddurchgang
- 42: Radialvorsprung des Stützelements
- 43: Längssteg des Stützelements

- 47: Bedienelement
- 48: Handbedienteil
- 49: Gehäuse
- 50: Drehzapfen
- 51: Drehlagerteil
- 52: Drehring

- 55: Drehverbindungsglied
- 56: Durchgangskanal
- 57: Kopplungseinrichtung
- 58: Kopplungshülse
- 59: Fluidraum
- 60: stirnseitige Öffnung der Kopplungshülse
- 61: Ringdichtung

- 65: erstes Fluidkopplungselement
- 65a: zweites Fluidkopplungselement
- 65b: Fluidkopplungselement
- 66: erster Eingangskanal
- 67: zweiter Eingangskanal
- 68: Ausgangskanal
- 69: Absaugschlauch
- 70: Fluiddichtung
- 71: Fluidleitung

- 75: Verbindungseinrichtung
- 76: Verbindungshülse
- 77: Verbindungselement
- 78: Haltevorsprung
- 79: Außenhülse
- 80: Anschlag
- 81: Aussparung des Kopfteils

- 85: Fußteil

- D: Drehachse
- F: Flüssigkeitsstrahl
- G: Gewebe
- GO: Gewebeoberfläche
- L: Längsachse
- P: Gewebepartikel
- Q: Querrichtung
- S: Absaugströmung
- X: Längsrichtung

## Patentansprüche

1. Vorrichtung (15) zum Abtragen von Gewebepartikeln (P) von einem Gewebe (G), insbesondere von einer Gewebeoberfläche (GO), aufweisend:
- ein Kopfteil (16), das einen Innenraum (24) umschließt und eine Kopfteilöffnung (25) aufweist, durch die der Innenraum (24) von außerhalb des Kopfteils (16) zugänglich ist und die benachbart zu einer Auflagefläche (17) des Kopfteils (16) angeordnet ist, wobei die Auflagefläche (17) zur Auflage auf der Gewebeoberfläche (GO) eingerichtet ist,
- eine Düsenrohr (26) und/oder Düsenschlauch, das sich entlang einer Längsachse (L) mit Abstand zur Kopfteilöffnung (25) in den Innenraum (24) erstreckt und mehrere zur Kopfteilöffnung (25) hin ausgerichtete Düsenöffnungen (30) aufweist,
- einen Zufuhrkanal (27), der fluidisch mit dem Düsenrohr (26) verbunden ist und der dazu eingerichtet ist, Flüssigkeit aus einer Flüssigkeitsquelle (29) zum Düsenrohr (26) zu leiten,
- einen Absaugkanal (34), der fluidisch mit dem Innenraum (24) verbunden ist und der dazu eingerichtet ist, Gewebepartikel (P) aus dem Innenraum (24) zu einer Partikelsammeleinrichtung (35) zu leiten.

2. Vorrichtung nach Anspruch 1, wobei der Absaugkanal (34) wenigstens eine Kanalmündung zum Innenraum (24) des Kopfteils (16) aufweist, die benachbart zum Düsenrohr (26) angeordnet ist, wobei insbesondere mehrere Kanalmündungen um das Düsenrohr (26) verteilt angeordnet sind.

3. Vorrichtung nach Anspruch 1 oder 2, wobei die Düsenöffnungen (30) dazu eingerichtet sind, die Flüssigkeitsstrahlen (F) parallel zueinander und/oder rechtwinkelig zur Kopfteilöffnung (25) auszurichten.

4. Vorrichtung nach einem der vorhergehenden Ansprüche, wobei die Düsenöffnungen (30) parallel zur Längsachse (L) entlang einer gemeinsamen Geraden angeordnet sind.

5. Vorrichtung nach einem der vorhergehenden Ansprüche, außerdem aufweisend eine mit dem Kopfteil (16) verbundene Außenhülle (18), die ein sich vom proximalen Ende bis zum distalen Ende der Außenhülle (18) erstreckendes Lumen (19) begrenzt.

6. Vorrichtung nach Anspruch 5, wobei in dem Lumen (19) ein Zufuhrschlauch (28) angeordnet ist, der den Zufuhrkanal (27) enthält und wobei vorzugsweise der Absaugkanal (34) in dem Lumen (19) angeordnet ist.

7. Vorrichtung nach einem der vorhergehenden Ansprüche, wobei das Kopfteil (16) um eine Drehachse (D) drehbar gelagert ist, die sich parallel zur Längsachse (L) oder entlang der Längsachse (L) erstreckt.

8. Vorrichtung nach Anspruch 7, wobei das Kopfteil (16) durch von außerhalb der Vorrichtung auf das Kopfteil (16) einwirkenden Kräfte passiv drehbar gelagert ist.

9. Vorrichtung nach Anspruch 5 oder 6 und nach Anspruch 7, wobei in dem Lumen (19) ein Drehverbindungsglied (55) angeordnet ist, das an einem distalen Ende drehfest mit dem Kopfteil (16) verbunden ist, das an einem proximalen Ende drehfest mit einem Bedienelement (47) verbunden ist, und das dazu eingerichtet ist ein Drehmoment vom Bedienelement (47) auf das Kopfteil (16) zu übertragen.

10. Vorrichtung nach Anspruch 9, wobei das Drehverbindungsglied (55) einen Durchgangskanal (56) aufweist, durch den sich der der Zufuhrkanal (27) und insbesondere der Zufuhrschlauch (28) erstreckt.

11. Vorrichtung nach Anspruch 9, wobei das Drehverbindungsglied (55) im Zufuhrkanal (27) und insbesondere im Zufuhrschlauch (28) angeordnet ist.

12. Vorrichtung nach einem der Ansprüche 7 bis 11 und nach Anspruch 6, wobei ein Drehlager (38) für die Drehbewegung zwischen der Außenhülle (18) und einem Lagerfortsatz (39) des Kopfteils (16) gebildet ist, wobei der Lagerfortsatz (39) vorzugsweise in das Lumen (19) der Außenhülle (18) hineinragt.

13. Vorrichtung nach Anspruch 12, wobei der Lagerfortsatz (39) wenigstens einen sich vom proximalen Ende des Lagerfortsatzes (39) bis zum Innenraum (24) des Kopfteils (16) erstreckenden Fluiddurchgang (41) aufweist.

14. Vorrichtung nach einem der vorhergehenden Ansprüche, wobei das Düsenrohr (26) relativ zum Kopfteil (16) unbeweglich angeordnet ist.

15. Anordnung aufweisend ein Endoskop (20) mit zumindest einem Arbeitskanal sowie eine Vorrichtung (15) nach einem der vorstehenden Ansprüche.

16. Verfahren zum Abtragen von Gewebepartikeln (P) von einem Gewebe (G), insbesondere von einer Gewebeoberfläche (GO) mittels einer Vorrichtung nach einem der Ansprüche 1 bis 14 oder einer Anordnung nach Anspruch 14, wobei das Verfahren umfasst:
- Anordnen der Auflagefläche (17) auf einer Gewebeoberfläche (GO),
- Zuführen von unter Druck stehender Flüssigkeit zum Düsenrohr (26), wodurch mehrere Flüssigkeitsstrahlen (F) aus den Düsenöffnungen (30) und durch die Kopfteilöffnung (25) auf das Gewebe (G) ausgestoßen werden, um Gewebepartikel (P) abzutragen, und
- Absaugen zumindest eines Teils der abgetragenen Gewebepartikel (P) aus dem Innenraum (24) des Kopfteils (16) über den Absaugkanal (34) zur Partikelsammeleinrichtung (35).
